(19) Europäisches Patentamt / European Patent Office / Office européen des brevets

(11) **EP 3 386 478 B1**

(12) **EUROPEAN PATENT SPECIFICATION**

(45) Date of publication and mention of the grant of the patent:
**13.03.2024 Bulletin 2024/11**

(21) Application number: **15848140.8**

(22) Date of filing: **31.12.2015**

(51) International Patent Classification (IPC):
**A61K 8/99** *(2017.01)*    **A61Q 19/00** *(2006.01)*
**A61K 8/11** *(2006.01)*    **A23L 33/135** *(2016.01)*
**A61Q 19/10** *(2006.01)*

(52) Cooperative Patent Classification (CPC):
**A61K 8/11; A23L 33/135; A61K 8/99; A61Q 19/10**

(86) International application number:
**PCT/LT2015/000009**

(87) International publication number:
**WO 2017/099559 (15.06.2017 Gazette 2017/24)**

(54) **COMPOSITION AND METHOD FOR INCREASE OF SURVIVAL AND STABILIZATION OF PROBIOTIC BACTERIA (PB) IN DETERGENT BASED COMPOSITIONS OF PERSONAL HYGIENE AND DOMESTIC PRODUCTS**

ZUSAMMENSETZUNG UND VERFAHREN ZUR ERHÖHUNG DER ÜBERLEBENSRATE UND STABILISIERUNG VON PROBIOTISCHEN BAKTERIEN (PB) IN REINIGERBASIERTEN ZUSAMMENSETZUNGEN FÜR KÖRPERHYGIENE- UND HAUSHALTSPRODUKTE

COMPOSITION ET PROCÉDÉ POUR AUGMENTER LA SURVIE ET LA STABILISATION DES BACTÉRIES PROBIOTIQUES (PB) DANS DES COMPOSITIONS À BASE DE DÉTERGENT DE PRODUITS D'HYGIÈNE PERSONNELLE ET DOMESTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.12.2015 LT 2015103**

(43) Date of publication of application:
**17.10.2018 Bulletin 2018/42**

(73) Proprietor: **UAB "Probiosanus"
08303 Vilnius (LT)**

(72) Inventors:
• **SIURKUS, Juozas**
  **LT-56433 Kaisiadorys (LT)**
• **LIESIENE, Jolanta**
  **LT-44093 Kaunas (LT)**
• **SIPAILIENÈ, Ausra**
  **LT-47267 Kaunas (LT)**
• **GRUZiNSKAITE, Aiste**
  **LT-60347 Raseiniu r. (LT)**

(74) Representative: **Klimaitiene, Otilija
AAA Law
A. Gostauto 40B
03163 Vilnius (LT)**

(56) References cited:
**WO-A1-2015/000972**

• **ZHAO MENG ET AL: "Microencapsulation ofLactobacillus acidophilusCGMCC1.2686: Correlation Between Bacteria Survivability and Physical Properties of Microcapsules", FOOD BIOPHYSICS, SPRINGER US, BOSTON, vol. 10, no. 3, 13 January 2015 (2015-01-13), pages 292-299, XP035520747, ISSN: 1557-1858, DOI: 10.1007/S11483-014-9389-5 [retrieved on 2015-01-13]**
• **PUSHPAK S. BORA: "Physicochemical Properties and Excipient Compatibility Studies of Probiotic Bacillus coagulans Spores", SCIENTIA PHARMACEUTICA, vol. 77, no. 3, 1 January 2009 (2009-01-01), pages 625-637, XP055300355, Austria ISSN: 0036-8709, DOI: 10.3797/scipharm.0904-01**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

   **(Cont. next page)**

• **TANZINA HUQ ET AL: "Encapsulation of Probiotic Bacteria in Biopolymeric System", CRITICAL REVIEWS IN FOOD SCIENCE AND NUTRITION, vol. 53, no. 9, 1 January 2013 (2013-01-01), pages 909-916, XP055291867, USA ISSN: 1040-8398, DOI: 10.1080/10408398.2011.573152**

**Description**

**Field of invention**

Present invention relates to a novel approach for preservation of probiotic bacteria by encapsulation in polymeric capsules with the aim to protect them from surfactants and other deteriorating compounds which are commonly used in personal care products including their final compositions.

**Background**

Probiotic bacteria are a group of non-pathogenic microorganisms, which are beneficial to human and animal health in various different mechanisms. The probiotic action could be related with the following mechanisms: (i) secretion of bactericides - metabolites, peptides and enzymes, pH modulation, biocompetition (ii), biofilm or barrier formation and immunomodulation (1 - Walker WA. (2008) Mechanisms of action of probiotics. Clin Infect Dis. 46 (Supplement 2): S87-S91 ). Probiotics may alter skin health and, for example, might be effective at preventing the development of atopic dermatitis (2- Yeşilova, Y., Çalka, Ö., Akdeniz, N., & Berktaş, M. (2012). Effect of Probiotics on the Treatment of Children with Atopic Dermatitis. Annals of Dermatology, 24(2), 189-193.), including prevention of skin diseases and improvement of skin condition (3 - Reid, G., Jass, J., Sebulsky, M. T., & McCormick, J. K. (2003). Potential Uses of Probiotics in Clinical Practice. Clinical Microbiology Reviews, 16(4), 658-672.). Some bacterial metabolites might act as antioxidants and thus reduces or prevents melanogenesis or other ROS including chemical agent related skin damage (4 - Tsao, R., Yang, R. and Young, J.C. (2003) Antioxidant isoflavones in orange, maclura pomífera (Raf.) Scheneid. J Agric Food Chem 51, 6445-6451., 5 - Wang, Y.C., Yu, R.C. and Chou, C-C. (2006) Antioxidative activities of soymilk fermented with lactic acid bacteria and bifidobacteria. Food Microbiol 23, 128-135). The health beneficial features of the probiotic or/and their metabolites for the skin is one of the factors which made them to be used as an active compounds in the compositions of personal hygiene or cosmetic products. The aim of probiotic bacteria within the cosmetic products is to diminish indications related with skin infections, pustules, skin fat balancing and reduction of dermatitis. One of examples is the patent EP 2306970 A1, which covers the invention of cosmetic compositions comprising lacto and/or bifido bacteria compounds in the range of 0,001-20 % (w/w) in combination with vitamins (B3, B5, B6, C, D, E, PP) niacine, carotenoids, polyphenols and minerals (Zn, Ca, Mg, Cu, Fe, I, Mn, Se, Cr), phytoestrogens, proteins, amino acids, mono- and polysaccharides, phytoterpenes, phytosterols and aminosugars. The *Lactobacillus paracasei* ST1 was used as the dominating bacterial in the described compositions. The patent application also describes the formats of probiotic based cremes, gels, powder and tablets (6- Patent application EP 2306970 A1).

Patent application FR 2889057 describes a cosmetic composition comprising lactic acid bacteria with unsaturated fatty acids and their esters (7- Patent application FR 2889057). Similarly, patent application WO 2006/07922 describes a cosmetic composition for sensitive skin, which is based on *Lactobacillus paracasei* or *casei* and *Bifidobacterium longum* or *Bifidobacterium lactis* bacteria in combination with other active ingredients (8 - WO 2006/07922). Applications WO 02/28402 and WO 03/070260 describes microbial based cosmetic compositions for sensitive skin with calming features and for protection of skin from damaging UV effects, respectively (9 patent application WO 02/28402, 10 and patent application WO 03/070260).

M. Zhao and others in the article "Microencapsulation of Lactobacillus acidophilus CGMCC1.2686: Correlation Between Bacteria Survivability and Physical Properties of Microcapsules" described correlation between lactic acid bacteria survivability and physical properties of microcapsules is critical to revealing the protecting mechanism of microcapsules. Five microcapsules with different mechanical strengths were used to encapsulate L. acidophilus CGMCC 1.2686. Mechanical measurements demonstrated that capsules with higher mechanical strengths are always more elastic, and images observed under electron scanning microscopy confirmed their denser structure.

"Physicochemical Properties and Excipient Compatibility studies of Probiotic Bacillus coagulans Spores" by P. S. Bora and others defined preformulation studies of probiotic Bacillus coagulans spores to aid designing of stable formulations. Bacillus coagulans spores were studied for hygroscopicity, resistance to compaction force, aqueous pH stability, and excipient compatibility. Aqueous suspension of Bacillus coagulans spores in buffer solutions of pH 1.2 to 8 showed rapid degradation with maximal stability in pH 6.8. Excipient compatibility studies showed reduced assay with citric acid monohydrate, meglumine and sodium starch glycolate. Bacillus coagulans spores were found to be sensitive to the conditions encountered in processing of pharmaceutical and food products.

The review "Encapsulation of Probiotic Bacteria in Biopolymeric System" by T. Huq and others focused on the method of encapsulation and the use of different biopolymeric system for encapsulation of probiotics. In order to protect the viability of the probiotic bacteria, several types of biopolymers such as alginate, chitosan, gelatin, whey protein isolate, cellulose derivatives were used for encapsulation and several methods of encapsulation such as spray drying, extrusion, emulsion have been reported. Biopolymers are the best effective materials for encapsulation of probiotics.

But when only one biopolymer is used for encapsulation, it does not exhibit appropriate effect on encapsulation. Mixture of biopolymers could have the best potential for the encapsulation of probiotics.

None of these IP protected compositions are referring to the probiotic or probiotic-based metabolite stability aspects in the direct contact with the chemical compounds of cosmetic products. The probiotic bacterial stability in various composition issues could be related with bactericide, bacteriostatic and lytic effects of cosmetic composition compounds especially surface active materials, preservatives, terpenes, alcohols and other. The surface active materials (SAMs) are the key compounds in the cosmetic and personal hygiene products, which could be for the functions of cleaning, moisturising, foaming or antifoaming and distributing/spreading and emulsifying. SAMs are water-soluble polymers due to so called amphophilic feature, where a molecule is composed of hydrophobic tale and hydrophilic moiety. In the water solutions SAMs are forming micelles - structured units due to the hydrophobic tail, which is excluding polar phase and hydrophilic residue which is oriented to the solute. According to the features of the molecules the SAMs are subdivided into anionic, cationic and non-polar (11- Surface-Active Agents: Advances in Research and Application: 2011 Edition. Edited by Ashton Acto). The most powerful cell lysis potential of bacterial cells is possessing the strong, polar SAMs which action of mechanism is based the interaction with bacterial membrane hydrophobic and hydrophilic patches, respectively. As the result of this interaction the bacterial cell membranes are ruptured resulting in the cell lysis and release of intracellular content - soluble proteins, ribosomes, DNA and RNA, into the environment (12 - Vasily N. Danilevich, Lada E. Petrovskaya, Eugene V. Grishin (2008) A Highly Efficient Procedure for the Extraction of Soluble Proteins from Bacterial Cells with Mild Chaotropic Solutions. Chem. Eng. Technol. 31, 6: 904-910). The bacterial stability against various physical and chemical factors can be increased by encapsulation into the various polymer capsules or immobilization into surfaces of various solid particles (13 - Riaz QU, Masud T.Recent trends and applications of encapsulating materials for probiotic stability. (2013) Crit Rev Food Sci Nutr. 53(3):231-44). There are several technologies, which are used for bacterial encapsulation: spray drying, extrusion and emulsification. The formed particles can be further stabilized by lyophilisation. The materials, which could be used for bacterial encapsulation are polysaccharides for example alginate, polysaccharides and lipids, and proteins. Depending on the encapsulation methodology and materials bacterial several main types of polymer capsules could be obtained - reservoir (bacterial cells are in the suspension, inside the capsule cavity) embedded inside the capsule polymer matrix and coated with polymer matrix. In addition, combinations of different polymers and encapsulation methodologies allow varying capsule sizes in range of 3 $\mu$m -5 mm. Finally, the bacterial cells release from capsules could be obtained by temperature treatment, moisture change, pH shifts, enzymatic treatment and exposure with mechanical force. The aim of excising bacterial encapsulation approaches is to protect probiotic bacterial cells in the digestion track of the human and animals against various hush factors conditions -usually extremely low pH (13 - Riaz QU, Masud T.Recent trends and applications of encapsulating materials for probiotic stability. (2013) Crit Rev Food Sci Nutr. 53(3):231-44).

## Summary of invention

Probiotic bacteria (PB) are well known group of microorganisms for the beneficial effects on human health. Thus, it is widely used as food and cosmetic, personal hygiene supplements (C/PHS).

PB positive action within the compositions of cosmetics could be related with the complementation of human microbiome, production of various anti-oxidative, moisturizing, biocide activity possessing compounds or other fermentation products which are directly or indirectly affecting human skin or other organ systems.

Gram positive PB and/or PB bacterial metabolites are supplemented into detergent based personal care or domestic hygiene compositions with the aim to improve product functional features. However, as defined in claim 8 the key compounds of (C/PHS): surface active materials (detergents: cationic - Sodium laureth sulfate (SLS), Sodium Cocoyl Glutamate, amphoteric: benzalkonium chloride, Cetrimonium chloride, Cocamidopropyl betaine, cocoamphoacetate, sodium cocoyl glycinate, non- ionic: diethanolamine and Coco-glucoside), conserving materials, terpenes, are directly acting on the PB as biocides, thus over the incubation time in these compositions, e.g. during product storage and usage the probiotic bacteria becoming inactivated - killed, resulting in the loss of the product composition and function. In addition, the metabolites of PB bacteria could interact with product compounds, could be exposed to UV light and/or oxidized and therefore chemically altered and/or inactivated.

We have used and approach for protection of probiotic bacteria, against the compounds of detergent based personal care or domestic hygiene products comprising bactericidal surface active materials- cationic, amphoteric and non-ionic detergents, preserving materials, terpenes (fragrances) and complex compositions comprising materials of groups mentioned above. The PB protection mechanism is based on bacterial immobilization/encapsulation into polymer pectin/alginate or alginate capsules, as defined in claim 5, which polymeric barrier allows maintaining of stable bacterial viability in the detergent based personal care or domestic hygiene products.

The PB protection mechanism, as defined in claim 1, is based on bacterial immobilization/encapsulation into polymer such as polysaccharides (e.g., pectin/alginate or alginate) or/and polysaccharides and lipids, or/and proteins capsules which polymeric barrier allows maintaining of stable bacterial viability in the detergent based personal care or domestic hygiene products.

As the example we demonstrated this invention using *Bacillus coagulans* bacteria and its metabolic compounds, by incubating known titters of encapsulated and non capsulated bacterial cells in various detergent compositions, personal care products.

We have experimentally showed that polymeric encapsulation results in the stabilization of alive PB titters, or decreases the bacterial inactivation during the 120 hours at the elevated temperatures - 37 °C, (which could be an equivalent to 120 days) of encap-bacterial incubation in the various detergent compositions within the personal hygiene products. At these experimental conditions, as defined in claim 9-10, we observed that viable encap-bacterial preservation is corresponding to survival of 60-90 % compared to the analogical compositions with non-encapsulated bacteria, where 60- 90 % of added bacteria to the composition were inactivated during the same incubation conditions.

**Description of Figures**

Figure 1. Provides results of survival of encapsulated and non-encapsulated *B. coagulans* bacteria after incubation in the solution comprising amphoteric surface active material - Cocoamido Propyl Betain.

Figure 2. Provides results of survival of encapsulated and non-encapsulated *B. coagulans* bacteria after incubation in the solutions comprising non-ionic surface active materials - 2 %, or Cocoglycozide-methyl oleate 3%, or Cocamide Diethanolamine or Cocoglycozide.

Figure 3. Provides results of survival of encapsulated and non-encapsulated *B. coagulans* bacteria after incubation in the solutions of Grapefruit seed extract, or d-limonene, or *Azadirachta indica* extract.

Figure 4 Provides survival results of *B. coagulans* bacteria in the polymer-encapsulated system and non-capsulated *B. coagulans* bacteria after incubation in the solution of non-ionic surface active materials comprising MILD HANDS WASHING-UP LIQUID WITH PROVITAMIN B5 (UAB Probiosanus) or PET SHAMPOO FOR LONG COAT B5 (UAB Probiosanus) or PET SHAMPOO FOR SHORT COAT (UAB Probiosanus).

**DETAILED DESCRIPTION OF THE INVENTION**

**Bacterial cell preparation**

For all experimental trails probiotic strain of *Bacillus coagulans* DSM 2311 was used which was obtained from DSMZ. Bacterial cell suspension was prepared by cultivation of *B. coagulans* cells in 100 mL of liquid nutrient broth medium (Oxoid) in 1000 mL Erlenmeyer flask, for 24 hours at 37°C while shaking at 180 rmp. Prior to encapsulation the cells were collected by centrifugation at 6000 rpm using Rotofix 32 A Hettich, then washed twice and re-suspended in sterile 0.9 % NaCl solution to obtain cell concentration of $10^{11}$ per mL.

**Bacterial cell encapsulation**

Encapsulation of *B. coagulans* cells was performed using emulsion formation approach. 200 mL of emulsion comprising of 40 % water phase and 60 % sunflower-seed oil was prepared and mixed to obtain homogenous consistency.

Water phase was composed of 2.4 % alginate and *B. coagulans* bacterial cells to obtain $10^9$-$10^{10}$ of bacterial cells per gram of capsules. Oil phase was composed of 99.5 % sunflower-seed oil with 1 % of POLYSORBATE 80. To obtain the emulsion, bacteria-alginate water phase was slowly introduced by pouring it into the oil phase solution and mixed for at least 20 minutes at room temperature. After emulsion was formed, sterile 5.5 % $CaCl_2$ solution was slowly added to obtain final concentration of 3 %. The reaction mixture was then incubated at the room temperature for several hours. During incubation the solution was exposed to a UV light source to reduce possibility of microbial contamination. The obtained bacterial-cell-harbouring capsules were collected by centrifugation at low G and washed twice with sterile deionised water and stored in 0.1 % tryptone water at +4°C. The bacterial titre per mass of the capsules was obtained $10^{10}$ / g of wet weight.

For further experimentation, capsules were lyophilised for 24 h at -110°C in Christ ALPHA 2-4 LSC and stored at -20°C or -80°C. The size of the matured capsules was determined using Malvern Mastersizer 2000 and varied in the range of 0.2- 0.24 mm lyophilized (30 minutes after rehydration in water) and 0.2-0.260mm non-lyophilized, respectively.

**Analysis of encapsulated bacteria viability in detergent based solution.**

1 g of ~$10^{10}$ bacteria-containing lyophilized and rehydrated or fresh microcapsules was added to the fixed volume (100 mL) of either: detergent solution, cosmetic compound containing solution or final formulation detergent based product and incubated at 37°C temperature. The samples were harvested at certain timepoints to evaluate bacterial survival. After sample harvest the bacterial capsules were washed twice with sterile water and added to a fixed volume of sterile 2% sodium citrate solution for liberation of encapsulated bacteria. The cell suspension was diluted from $10^1$ to $10^5$ fold and 100 $\mu$L of solution was plated on Petri dishes filled with Nutrient Broth solid medium. The survival rates [%] of capsulated and non-capsulated bacteria was estimated by comparing CFU averages at each sample point, from 3 replicas. See formulas:

$$N\ (CFU) = \left(\frac{CFU * Vfd}{Vp}\right) * D$$

N- estimated CFU;
CFU - sum of calculated colonies from each evaluated Petri plates
$V_p$ - plated volume
$Vfd$- final dilution volume
D - dilution fold

$$\text{Survival of bacteria } [\%] = N_{(1+n)}/N_1 * 100$$

$N_1$ - bacterial CFU's from first timepoint sample
$N_{(1+n)}$ - bacterial CFU's from following timepoint sample

**Embodiments of invention**

**Example 1.**

1 g of $10^{10}$ bacteria-containing rehydrated or fresh microcapsules was added to the fixed volume (100 mL) of detergent solution and incubated for 120 hours at 37°C temperature. A parallel experiment was performed with non-capsulated cells (control group). After the sample harvest the bacterial capsules were washed twice with sterile water and added to the fixed volume of sterile 2% sodium citrate solution for liberation of encapsulated bacteria. The cell suspension was diluted $10^1$-$10^5$ fold and 100 $\mu$L plated on replicates of Nutrient Broth solid medium. The survival rates [%] of capsulated and non-capsulated bacteria were estimated as described in the section - **Analysis of encapsulated bacteria viability in detergent based solution**, by comparing CFU averages at each sample time point from 3 replicas.

**a. Non-capsulated and encapsulated bacteria survival in solution of amphoteric surface-active material - cocoamide propyl betaine (CAPB) 6.7% (stock concentration 99%, BASF).**

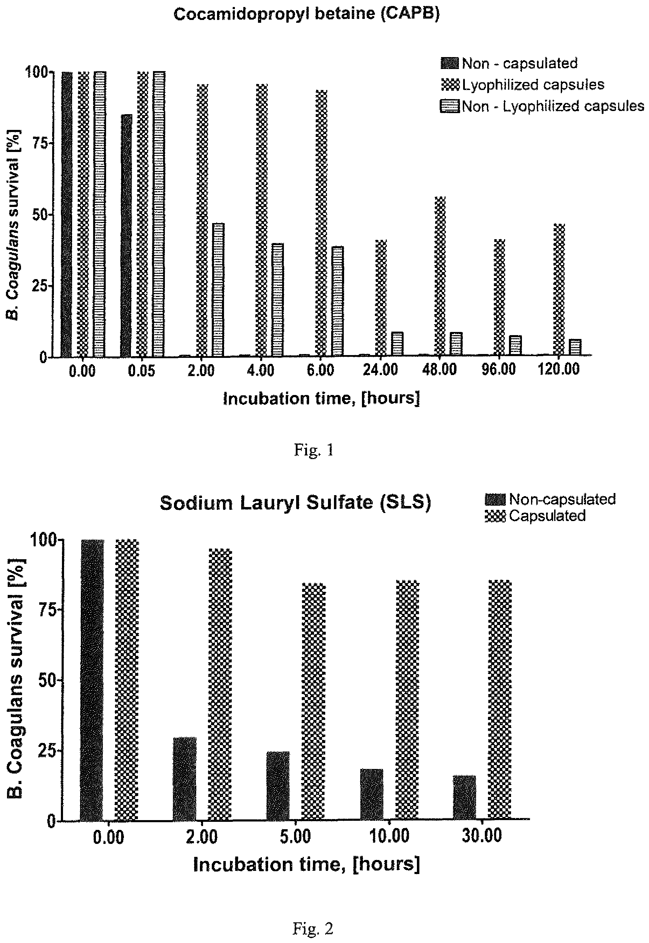

The experiments revealed that 97% of non-capsulated bacteria (starting titers) were inactivated after 0.5h due to the exposure to the CAPB, while ~50 % and ~35 % survived in lyophilized and non-lyophilized capsules after incubation for 120h at 37 °C, respectively (Figure 1).
**Conclusions:** encapsulation effectively preserved *B. coagulans* bacteria in solution of amphoteric surface active material - cocoamido propyl betain while this compound inactivated the same strain of non-capsulated bacteria.

**b. Non-capsulated and lyophilized encapsulated bacteria survival in anionic surface active material - 6.7 % sodium lauryl sulphate (SLS) (stock concentration 98 %, BASF) solution.**

The experiments revealed that 80% of non-capsulated bacteria were inactivated due to exposure to SLS, while -80% survived in lyophilized capsules after incubation for 120 h at 37°C (Figure_2).
Encapsulation effectively preserved *B. coagulans* bacteria in SLS solution while this compound inactivated the same strain of non-capsulated bacteria.

**c.Non-capsulated and encapsulated lyophilized bacteria survival in non-ionic surface active materials: 2 % cocoglycozide-methyl oleate (stock concentration 98 %, BASF), 3%, cocamide diethanolamine (stock concentration 98%, BASF) and 7% cocoglycozide (stock concentration 98 % BASF) (Panel C) solutions.**

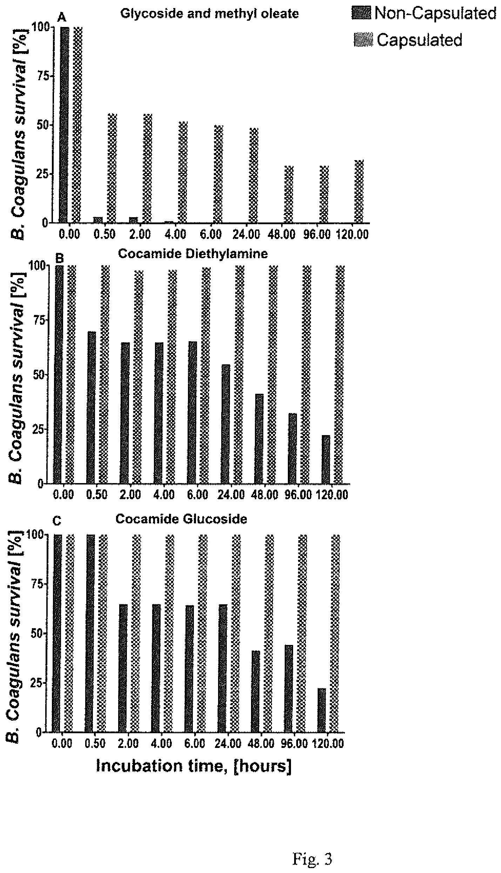

The experiments revealed that 98% of non-capsulated bacteria were inactivated due to the exposure to co-cocoglycozide-methyl oleate after 0.5h incubation, while ~50% survived for 120h in lyophilized capsules (Panel A); 75% of non-capsulated bacteria were inactivated due to the exposure to cocamide diethanolamine (Panel B) and cocoglycozide (Panel C), while >95% survived in lyophilized capsules after incubation for 120h at 37°C (Panel B and C) Figure_3.

Encapsulation effectively preserved *B. coagulans* bacteria in compositions comprising natural non-ionic surface active materials while these compounds inactivated the same strain of non-capsulated bacteria.

**Example 2.**

1 g of $10^{10}$ bacteria containing lyophilized - rehydrated microcapsules was added to the fixed volume (100 mL) of solutions of: Grapefruit seed extract d-limonene, or *Azadirachta indica* extract and incubated for 120 hours at 37 °C temperature. A parallel experiment was performed with non-capsulated cells. After sample harvest the bacterial capsules were washed twice with sterile water and added to the fixed volume of sterile 2% sodium citrate solution for liberation of encapsulated bacteria. The cell suspension was diluted $10^1$-$10^5$ fold and 100 μL of solution was plated on replicates of Nutrient Broth solid medium. The survival rates [%] of capsulated and non-capsulated bacteria were estimated as described in the section - **Analysis of encapsulated bacteria viability in detergent based solution**, by comparing CFU averages at each sample point, from 3 replicas.

a. **Non-capsulated and encapsulated lyophilized bacteria survival in solution of 0.6 % preservative - Grapefruit seed extract ((*Citrus paradisi*) - stock concentration 98 %, LemonConcentrate) (panel A)), 0.7 % terpene, d-limonene (stock concentration 99 %, LemonConcentrate) (panel B) or 5 % *Azadirachta indica* extract (stock concentration, 90%, JSC"Baltkos") (Panel C).**

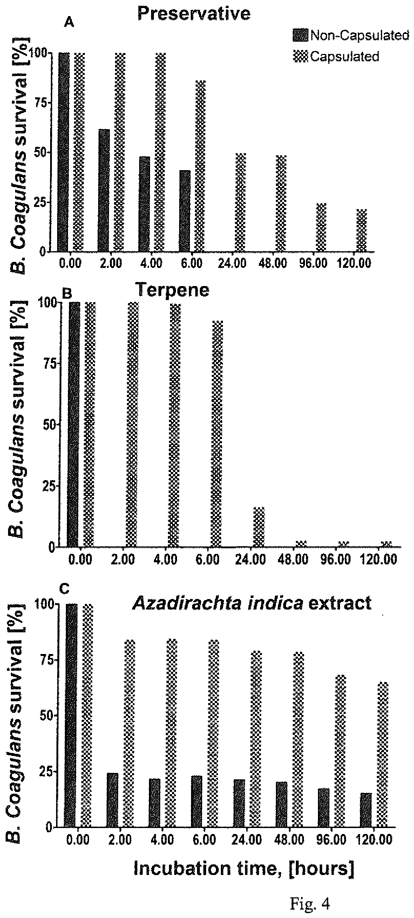

The experiments revealed that 98% of non-capsulated *B. Coagulans* bacteria were inactivated due to exposure to the preservative - Grapefruit seed extract after 6h of incubation inactivated instigated *B. Coagulans* bacteria >90 % , while >50 % survived for 24h and >35 % after 120h in lyophilized capsules (Panel A); 100 % of non-capsulated *B. Coagulans* bacteria were inactivated in 2 hours due to the exposure to terpenes (Panel B) and 75 % due to the action of *Azadirachta indica* extract (Panel C), while ~80 % of *B. Coagulans* survived in lyophilized capsules after incubation at 37°C for 6h and 75% for 120h, respectively (Figure 4 (Panel B and C)).

Encapsulation effectively preserved *B. coagulans* bacteria in compositions comprising natural seed-extract based preservative, terpenes and *Azadirachta indica* extract while these compounds inactivated the same strain of non-capsulated bacteria.

The examples 1 and 2 are present for illustration purposes only.

**Example 3.**

In this experiment, as detergent containing formulations were used three commercial products, all of UAB Probiosanus: MILD LIQUID HAND WASH WITH PROVITAMIN B5 with the composition listed in the Table 1; PET SHAMPOO FOR LONG COAT B5 with the composition listed in Table 2 and PET SHAMPOO FOR SHORT COAT UAB Probiosanus) with the composition listed in Table 3.

1 g of $10^{10}$ bacteria containing lyophilized - rehydrated microcapsules was added to the fixed volume (100 mL) of detergent containing formulation and incubated for 120 hours at 37°C temperature. A parallel experiment was performed with non-capsulated cells. After sample harvest the bacterial capsules were washed twice with sterile water and added to the fixed volume of sterile 2% sodium citrate solution for liberation of encapsulated bacteria. The cell suspension was diluted $10^1$-$10^5$ fold and 100 μL of solution was plated on replicates of Nutrient Broth solid medium. The survival rates [%] of capsulated and non-capsulated bacteria were estimated as described in the section - **Analysis of encapsulated bacteria viability in detergent based solution,** by comparing CFU averages at each sample point, from 3 replicas.

**Results.** Non-capsulated and encapsulated bacteria survival in lyophilized alginate micro capsules (diameter -0.24 mm) in solution of non-ionic surface active materials comprising MILD LIQUID HAND WASH WITH PROVITAMIN B5.

Table 1. **MILD LIQUID HAND WASH WITH PROVITAMIN B5 (UAB Probiosanus)**

| Compound | CAS Nb. | Mass, [%] |
|---|---|---|
| Sodium laureth sulfate (SLS concentration 70%) | 68891-38-3 | 6,4 |
| Alkyl (C8-C10) polyglycozide (concentration 70%) | 68515-73-1 | 3,6 |
| (Cocamide DEA, concentration 85%) | 68603-42-9 | 2,8 |
| Glycerol | 56-81-5 | 4 |

(continued)

| Compound | CAS Nb. | Mass, [%] |
|---|---|---|
| Orange terpenes | 5989-27-5 | 0,35 |
| Fragrance | 233799 | 0,1 |
| D-pantenol | - | 0,4 |
| Ethyl hydroxyethyl cellulose (EHEC) | 9004-58-4 | 0,9 |
| Preservative (Orange terpenes) | 26172-55-4 2682-20-4 100-51-6 | 0,2 |
| Citric acid | 5949-29-1 | 0,015 |

Table 2. **PET SHAMPOO FOR LONG COAT B5** (UAB Probiosanus)

| Compound | CAS Nb. | Mass, [%] |
|---|---|---|
| Sodium Cocoyl Glutamate (concentration 99 % ) | 68187-30-4 | 10 |
| Coco Glucoside (concentration 99%) | 110615-47-9, 68515-73-1 | 7,0 |
| Coco Betaine (concentration 99%) | 66455-29-6 | 5,0 |
| Neem extract (*Azadirachta indica*) | 84696-25-3/ 90063-92-6 | 1 |
| Chamomila *recutita* extract | - | 1 |
| Nettle extract | 84012-40-8 | 1 |
| Xanthan gum | 11138-66-2 | 0,75 |
| Lactic acid/80 | 598-82-3 | 0,5 |
| Preservative ACNIBIO C PLUS | 90045-43-5 | 0,06 |

Table 3. **PET SHAMPOO FOR SHORT COAT B5** (UAB Probiosanus)

| Compound | CAS Nb. | Mas s, [%] |
|---|---|---|
| Caprylyl Capryl Glucoside (concentration 99 %) | 68515-73-1 | 5,38 |
| Sodium Cocoyl Glutamate (concentration 99%) | 68187-30-4 | 4 |
| Coco Betaine ( concentration 99%) | 66455-29-6 | 6,7 |
| Glyceryl Oleate (and) Coco Glucoside | 77-92-9, 141464-42-8 | 3,.85 |
| Neem extract (*Azadirachta indica*) | 84696-25-3/ 90063-92-6 | 1 |
| *Chamomila recutita* extract | - | 1 |
| Nettle (Urtica dioica) extract | 84012-40-8 | 1 |
| Xanthan gum | 11138-66-2 | 0,75 |
| Preservative - citrus seed extract | 90045-43-5 | 0,06 |

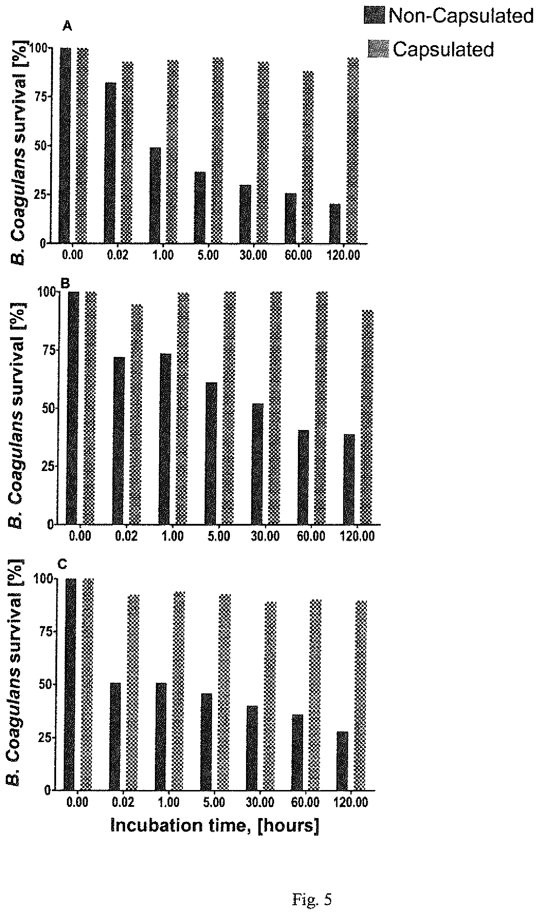

The experiments revealed that 80 % of non-capsulated bacteria were inactivated after incubation for 120 h at 37 ° C due to the exposure to MILD LIQUID HAND WASH WITH PROVITAMIN B5 comprising major bacteriocidic components (i) SAMs: Sodium laureth sulfate, Alkyl (C8-C10) polyglycozide and Cocamide DEA (ii) terpenes: Orange terpenes, while > 95 % survived in lyophilized capsules (Panel A); 60 % of non-capsulated bacteria were inactivated after incubation for 120 h at 37 ° C due to the exposure to PET SHAMPOO FOR LONG COAT B5 comprising (i) SAMs: Sodium Cocoyl Glutamate, Coco Glucoside, Coco betain (ii) Plant extracts: Neem extract (*Azadirachta indica*), Nettle (*Urtica Dioica*) and (iii) Preservative: ACNIBIO C PLUS which is based on ascorbic acid (vitamin C) and bioflavonoids from citric fruit (vitamin P): (Panel B) and ~ 75 % PET SHAMPOO FOR SHORT COAT (Panel C) comprising (i) SAMs: Sodium Cocoyl Glutamate, Coco Glucoside, Coco betain and : Glyceryl Oleate (and) Coco Glucoside, (ii) Plant extracts Neem extract (*Azadirachta indica*) and *Chamomila recutita*, Nettle (*Urtica dioica* ), (iii) Preservative based on citrus seed extract, while ~> 90 % of *B. Coagulans* survived in lyophilized capsules after incubation in the both for 120 h at 37 °C (Figure_5 (Panel B and C)).

**Conclusions:** encapsulation effectively preserved *B. coagulans* bacteria in the personal hygiene/care product compositions comprising combinations of anionic and/or non-ionic surface and/or amphoteric surface active materials with natural seed-extracts and preservative based on terpenes and vitamins, while the same compositions inactivated non-capsulated *B. coagulans* bacteria.

**Literature as cited:**

1. Walker WA. (2008) Mechanisms of action of probiotics. Clin Infect Dis. 46 (Supplement 2): S87-S91

2. YeŞilova, Y., Çalka, Ö., Akdeniz, N., & BerktaŞ, M. (2012). Effect of Probiotics on the Treatment of Children with Atopic Dermatitis. Annals of Dermatology, *24*(2), 189-193.

3. Reid, G., Jass, J., Sebulsky, M. T., & McCormick, J. K. (2003). Potential Uses of Probiotics in Clinical Practice. Clinical Microbiology Reviews, 16(4), 658-672.

4. Tsao, R., Yang, R. and Young, J.C. (2003) Antioxidant isoflavones in orange, maclura pomífera (Raf.) Scheneid. J Agric Food Chem 51, 6445-6451.

5. Wang, Y.C., Yu, R.C. and Chou, C-C. (2006) Antioxidative activities of soymilk fermented with lactic acid bacteria and bifidobacteria. Food Microbiol 23, 128-135.

6. Patent application EP 2306970 A1

7. Patent application FR 2889057

8. Patent application WO 2006/07922

9. Patent application WO 02/28402

10. Patent application WO 03/070260

11. Surface-Active Agents: Advances in Research and Application: 2011 Edition. Edited by Ashton Acton

12. Vasily N. Danilevich, Lada E. Petrovskaya, Eugene V. Grishin (2008) A Highly Efficient Procedure for the Extraction of Soluble Proteins from Bacterial Cells with Mild Chaotropic Solutions. Chem. Eng. Technol. 31, 6: 904-910

13. Riaz QU, Masud T.Recent trends and applications of encapsulating materials for probiotic stability. (2013) Crit Rev Food Sci Nutr. 53(3):231-44.

14. Patent aplication WO2015000972A1

15. Patent aplication WO2015019307A1

16. Patent aplication US20080107699A1

17. Patent aplication WO2004035885A1

18. WO2013188626A2

19. US20140065218A1

20. US9157054B2

21. WO2010045541A1

22. US20150071977A1

23. M. Zhao, F. Qu, S. Cai, Y. Fang, K. Nishinari, G.O. Phillips, F. Jiang, Microencapsulation of lactobacillus acidophilus CGMCC1. 2686: correlationbetween bacteria survivability and physical properties of microcapsules, Food-Biophys. 10 (2015) 292-299, https://doi.org/10.1007/s11483-014-9389-5.

24. Bora, P.S.; Puri, V.; Bansal, A.K. Physicochemical Properties and Excipient Compatibility studies of Probiotic Bacillus coagulans Spores. Sci. Pharm. 2009, 77, 625-638. https://doi.org/10.3797/scipharm.0904-01

25. Huq, T., Khan. A., Khan, R.A., Riedl, B., Lacroix, M. Encapsulation of Probiotic Bacteria in Biopolymeric System, Critical Reviews in Food Science and Nutrition, 2013, 53:9, 909-916, DOI: 10.1080/10408398.2011.573152

**Claims**

1.  Liquid composition **characterized in that** the composition comprises:

polymeric capsules with encapsulated viable Gram positive or/and viable Gram negative bacterial cells, wherein said polymeric capsules comprises polysaccharides or/and polysaccharides and lipids, or/and proteins; and at least one bactericidal surface active material or combination thereof, wherein the bactericidal surface active materials are selected from cocoglycozide-methyl oleate, cocamide diethanolamine, cocoglycozide, cocoamide propyl betaine and sodium lauryl sulphate; and

at least one of phyto-extracts, or/and terpenes or/and alcohols, or/and fatty acids, or/and organic acids, and/or vitamins, and/or bacteriocidic/static preservative, and fragrances, and thickeners.

2. Liquid composition of claim 1, **characterised in that** polymeric capsules size ranges from 0,2 mm to 5 mm.

3. Liquid composition according to any of claims 1-2, **characterised in that** encapsulated viable Gram positive or/and viable Gram negative bacterial cells count is from 1 to $1*10^9$ per polymer capsule and from 1 to $1*10^{12}$ of viable cells per gram of capsules wet weight.

4. Liquid composition according to any of claims 1-3, wherein said thickeners are selected from Xanthan gum and Celluloses in combination or separately.

5. Liquid composition according to any of claims 1-4, **characterized in that** said polymer comprises alginate.

6. Liquid composition according to any of claims 1-5, **characterized in that** said preservative is grapefruit seed extract or/and terpene or/and d-limonene.

7. Liquid composition according to any of claims 1-6, **characterized in that** said fragrancies are selected from plant fruiting body, fruit or seed extract/oil from *Azadirachta indica*, *Urtica dioica*, *Chamomila recutita*, *Cannabis sativa* extract.

8. Liquid composition according to any of claims 1-5, **characterized in that** the composition consists of 1 g of polymeric capsules with encapsulated viable bacterial cells in admixture with 100 ml of combination of bactericidal surface active materials the components ratio of the latter being as follows:

| Compound | CAS Nb. | Mass, [%] |
|---|---|---|
| Sodium laureth sulfate (SLS 70%) | 68891-38-3 | 6,4 |
| Alkyl (C8-C10) polyglycozide (70%) | 68515-73-1 | 3,6 |
| (Cocamide DEA 85%) | 68603-42-9 | 2,8 |
| Glycerol | 56-81-5 | 4 |
| Orange terpenes | 5989-27-5 | 0,35 |
| Fragrance | 233799 | 0,1 |
| D-panthenol | - | 0,4 |
| Ethyl hydroxyethyl cellulose (EHEC) | 9004-58-4 | 0,9 |
| Preservative (Orange terpenes) | 26172-55-4 2682-20-4 100-51-6 | 0,2 |
| Citric acid | 5949-29-1 | 0,015 |

9. Liquid composition according to any of claims 1-5 **characterized in that** the composition consists of 1 g of polymeric capsules with encapsulated viable bacterial cells in admixture with 100 ml of combination of bactericidal surface active materials the components ratio of the latter being as follows:

| Compound | CAS Nb. | Mass, [%] |
|---|---|---|
| Sodium Cocoyl Glutamate | 68187-30-4 | 10 |

(continued)

| Compound | CAS Nb. | Mass, [%] |
|---|---|---|
| Coco Glucoside | 110615-47-9, 68515-73-1 | 7,0 |
| Coco Betaine | 66455-29-6 | 5,0 |
| Neem extract (*Azadirachta indica*) | 84696-25-3/ 90063-92-6 | 1 |
| *Chamomila recutita* extract | - | 1 |
| Nettle extract | 84012-40-8 | 1 |
| Xanthan gum | 11138-66-2 | 0,75 |
| Lactic acid/80 | 598-82-3 | 0,5 |
| Preservative ACNIBIO C PLUS | 90045-43-5 | 0,06 |

**10.** Liquid composition according to any of claims 1-5 **characterized in that** the composition consists of 1 g of polymeric capsules with encapsulated viable bacterial cells in admixture with 100 ml of combination of bactericidal surface active materials the components ratio of the latter being as follows:

| Compound | CAS Nb. | Mass, [%] |
|---|---|---|
| Caprylyl Capryl Glucoside | 68515-73-1 | 5,38 |
| Sodium Cocoyl Glutamate | 68187-30-4 | 4 |
| Coco Betaine | 66455-29-6 | 6,7 |
| Glyceryl Oleate (and) Coco Glucoside | 77-92-9, 141464-42-8 | 3,85 |
| Neem extract (*Azadirachta indica*) | 84696-25-3/ 90063-92-6 | 1 |
| *Chamomila recutita* extract | - | 1 |
| Nettle (*Urtica dioica*) extract | 84012-40-8 | 1 |
| Xanthan gum | 11138-66-2 | 0,75 |
| Preservative - citrus seed extract | 90045-43-5 | 0,06 |

**Patentansprüche**

**1.** Flüssige Zusammensetzung, **dadurch gekennzeichnet, dass** die Zusammensetzung Folgendes umfasst:

Polymerkapseln mit verkapselten lebensfähigen grampositiven oder/und lebensfähigen gramnegativen Bakterienzellen, wobei die Polymerkapseln Polysaccharide oder/und Polysaccharide und Lipide oder/und Proteine umfassen; und
mindestens ein bakterizides oberflächenaktives Material oder eine Kombination daraus, wobei die bakteriziden oberflächenaktiven Materialien ausgewählt sind aus Cocoglycosidmethyloleat, Cocamiddiethanolamin, Cocoglycosid, Cocoamidpropylbetain und Natriumlaurylsulfat; und
mindestens eines von Phytoextrakten oder/und Terpenen oder/und Alkoholen oder/und Fettsäuren oder/und organischen Säuren und/oder Vitaminen und/oder bakteriziden/statischen Konservierungsstoffen und Duftstoffen und Verdickungsmitteln.

**2.** Flüssige Zusammensetzung nach Anspruch 1, **dadurch gekennzeichnet, dass** die Größe der Polymerkapseln im Bereich von 0,2 mm bis 5 mm liegt.

**3.** Flüssige Zusammensetzung nach einem der Ansprüche 1-2, **dadurch gekennzeichnet, dass** die Anzahl der verkapselten lebensfähigen grampositiven oder/und lebensfähigen gramnegativen Bakterienzellen 1 bis $1*10^9$ pro Polymerkapsel und 1 bis $1*10^{12}$ lebensfähige Zellen pro Gramm Nassgewicht der Kapseln beträgt.

4. Flüssige Zusammensetzung nach einem der Ansprüche 1-3, wobei die Verdickungsmittel aus Xanthangummi und Cellulosen in Kombination oder separat ausgewählt sind.

5. Flüssige Zusammensetzung nach einem der Ansprüche 1-4, **dadurch gekennzeichnet, dass** das Polymer Alginat umfasst.

6. Flüssige Zusammensetzung nach einem der Ansprüche 1-5, **dadurch gekennzeichnet, dass** das Konservierungsmittel Grapefruitsamenextrakt oder/und Terpen oder/und D-Limonen ist.

7. Flüssige Zusammensetzung nach einem der Ansprüche 1-6, **dadurch gekennzeichnet, dass** die Duftstoffe ausgewählt sind aus pflanzlichem Fruchtkörper, Frucht- oder Samenextrakt/Öl aus *Azadirachta indica*, *Urtica dioica*, *Chamomilla recutita,* Extrakt aus *Cannabis sativa.*

8. Flüssige Zusammensetzung nach einem der Ansprüche 1-5, **dadurch gekennzeichnet, dass** die Zusammensetzung aus 1 g Polymerkapseln mit verkapselten lebensfähigen Bakterienzellen in Beimischung mit 100 ml einer Kombination aus bakteriziden oberflächenaktiven Materialien besteht, wobei das Komponentenverhältnis der Letzteren wie folgt lautet:

| Verbindung | CAS-Nr. | Masse, [%] |
|---|---|---|
| Natriumlaurethsulfat (SLS 70 %) | 68891-38-3 | 6,4 |
| Alkyl(C8-C10)polyglycosid (70 %) | 68515-73-1 | 3,6 |
| (Cocamid DEA 85 %) | 68603-42-9 | 2,8 |
| Glycerin | 56-81-5 | 4 |
| Orangenterpene | 5989-27-5 | 0,35 |
| Duftstoff | 233799 | 0,1 |
| D-Panthenol | - | 0,4 |
| Ethylhydroxyethylcellulose (EHEC) | 9004-58-4 | 0,9 |
| Konservierungsmittel (Orangenterpene) | 26172-55-4 2682-20-4 100-51-6 | 0,2 |
| Citronensäure | 5949-29-1 | 0,015 |

9. Flüssige Zusammensetzung nach einem der Ansprüche 1-5, **dadurch gekennzeichnet, dass** die Zusammensetzung aus 1 g Polymerkapseln mit verkapselten lebensfähigen Bakterienzellen in Beimischung mit 100 ml einer Kombination aus bakteriziden oberflächenaktiven Materialien besteht, wobei das Komponentenverhältnis der Letzteren wie folgt lautet:

| Verbindung | CAS-Nr. | Masse, [%] |
|---|---|---|
| Natriumcocoylglutamat | 68187-30-4 | 10 |
| Cocoglucosid | 110615-47-9, 68515-73-1 | 7,0 |
| Cocobetain | 66455-29-6 | 5,0 |
| Neemextrakt (*Azadirachta indica*) | 84696-25-3/ 90063-92-6 | 1 |
| Extrakt aus *Chamomilla recutita* | - | 1 |
| Brennnesselextrakt | 84012-40-8 | 1 |
| Xanthangummi | 11138-66-2 | 0,75 |
| Milchsäure/80 | 598-82-3 | 0,5 |

(fortgesetzt)

| Verbindung | CAS-Nr. | Masse, [%] |
|---|---|---|
| Konservierungsmittel ACNIBIO C PLUS | 90045-43-5 | 0,06 |

**10.** Flüssige Zusammensetzung nach einem der Ansprüche 1-5, **dadurch gekennzeichnet, dass** die Zusammensetzung aus 1 g Polymerkapseln mit verkapselten lebensfähigen Bakterienzellen in Beimischung mit 100 ml einer Kombination aus bakteriziden oberflächenaktiven Materialien besteht, wobei das Komponentenverhältnis der Letzteren wie folgt lautet:

| Verbindung | CAS-Nr. | Masse, [%] |
|---|---|---|
| Caprylylcaprylglucosid | 68515-73-1 | 5,38 |
| Natriumcocoylglutamat | 68187-30-4 | 4 |
| Cocobetain | 66455-29-6 | 6,7 |
| Glyceryloleat (und) Cocoglucosid | 77-92-9, 141464-42-8 | 3,85 |
| Neemextrakt (*Azadirachta indica*) | 84696-25-3/ 90063-92-6 | 1 |
| Extrakt aus *Chamomilla recutita* | - | 1 |
| Extrakt aus der Brennnessel (*Urtica dioica*) | 84012-40-8 | 1 |
| Xanthangummi | 11138-66-2 | 0,75 |
| Konservierungsmittel - Zitrussamenextrakt | 90045-43-5 | 0,06 |

## Revendications

**1.** Composition liquide **caractérisée en ce que** la composition comprend :

des capsules polymères contenant des cellules bactériennes Gram positives viables ou/et Gram négatives viables encapsulées, dans laquelle lesdites capsules polymères comprennent des polysaccharides ou/et des polysaccharides et des lipides, ou/et des protéines ; et
au moins un matériau tensioactif bactéricide ou une combinaison de ceux-ci, dans laquelle les matériaux tensioactifs bactéricides sont choisis parmi le cocoglycoside-oléate de méthyle, la cocamide diéthanolamine, le cocoglycoside, la cocoamide propylbétaïne et le laurylsulfate de sodium ; et
au moins l'un parmi des phyto-extraits, ou/et des terpènes ou/et des alcools, ou/et des acides gras, ou/et des acides organiques, et/ou des vitamines, et/ou un conservateur bactéricide/statique, et des parfums et des épaississants.

**2.** Composition liquide selon la revendication 1, **caractérisée en ce que** la taille des capsules polymères va de 0,2 mm à 5 mm.

**3.** Composition liquide selon l'une quelconque des revendications 1 et 2, **caractérisée en ce que** le nombre de cellules bactériennes Gram positives viables ou/et Gram négatives viables encapsulées est de 1 à $1*10^9$ par capsule polymère et de 1 à $1*10^{12}$ cellules viables par gramme de poids de capsules à l'état humide.

**4.** Composition liquide selon l'une quelconque des revendications 1 à 3, dans laquelle lesdits épaississants sont choisis parmi la gomme xanthane et les celluloses en combinaison ou séparément.

**5.** Composition liquide selon l'une quelconque des revendications 1 à 4, **caractérisée en ce que** ledit polymère comprend un alginate.

**6.** Composition liquide selon l'une quelconque des revendications 1 à 5, **caractérisée en ce que** ledit conservateur est un extrait de pépins de pamplemousse ou/et un terpène ou/et le d-limonène.

**7.** Composition liquide selon l'une quelconque des revendications 1 à 6, **caractérisée en ce que** lesdits parfums sont choisis parmi un extrait/une huile de corps fructifère, de fruit ou de graine de plante provenant d'un extrait d'*Azadirachta indica*, d'*Urtica dioica*, de *Chamomilla recutita*, de *Cannabis sativa.*

**8.** Composition liquide selon l'une quelconque des revendications 1 à 5, **caractérisée en ce que** la composition est constituée de 1 g de capsules polymères contenant des cellules bactériennes viables encapsulées en mélange avec 100 ml d'une combinaison de matériaux tensioactifs bactéricides, le rapport des composants de ces dernières étant comme suit :

| Composé | N° CAS. | Masse,[%] |
|---|---|---|
| Lauryl éther sulfate de sodium (SLS 70 %) | 68891-38-3 | 6,4 |
| Alkylpolyglucoside (C8-C10) (70 %) | 68515-73-1 | 3,6 |
| (Cocamide DEA 85 %) | 68603-42-9 | 2,8 |
| Glycérol | 56-81-5 | 4 |
| Terpènes d'orange | 5989-27-5 | 0,35 |
| Parfum | 233799 | 0,1 |
| D-panthénol | - | 0,4 |
| Éthylhydroxyéthylcellulose (EHEC) | 9004-58-4 | 0,9 |
| Conservateur(Terpènes d'orange) | 26172-55-4 2682-20-4 100-51-6 | 0,2 |
| Acide citrique | 5949-29-1 | 0,015 |

**9.** Composition liquide selon l'une quelconque des revendications 1 à 5, **caractérisée en ce que** la composition est constituée de 1 g de capsules polymères contenant des cellules bactériennes viables encapsulées en mélange avec 100 ml d'une combinaison de matériaux tensioactifs bactéricides, le rapport des composants de ces dernières étant comme suit :

| Composé | N° CAS. | Masse,[%] |
|---|---|---|
| Cocoylglutamate de sodium | 68187-30-4 | 10 |
| CocoGlucoside | 110615-47-9, 68515-73-1 | 7,0 |
| CocoBétaïne | 66455-29-6 | 5,0 |
| Extrait de Neem (*Azadirachla indica*) | 84696-25-3/ 90063-92-6 | 1 |
| Extrait de *Camomilla recutita* | - | 1 |
| Extrait d'ortie | 84012-40-8 | 1 |
| Gomme xanthane | 11138-66-2 | 0,75 |
| Acide lactique/80 | 598-82-3 | 0,5 |
| Conservateur ACNIBIO C PLUS | 90045-43-5 | 0,06 |

**10.** Composition liquide selon l'une quelconque des revendications 1 à 5, **caractérisée en ce que** la composition est constituée de 1 g de capsules polymères contenant des cellules bactériennes viables encapsulées en mélange avec 100 ml d'une combinaison de matériaux tensioactifs bactéricides, le rapport des composants de ces dernières étant comme suit :

| Composé | N° CAS. | Masse, [%] |
|---|---|---|
| Caprylyl/Capryl Glucoside | 68515-73-1 | 5,38 |

| | | |
|---|---|---|
| Cocoylglutamate de sodium | 68187-30-4 | 4 |
| CocoBétaïne | 66455-29-6 | 6,7 |
| Oléate de Glycéryle (et) Coco Glucoside | 77-92-9, 141464- 42-8 | 3,85 |
| Extrait de Neem (*Azadirachla indica*) | 84696-25-3/ 90063-92-6 | 1 |
| Extrait de *Camomilla recutita* | - | 1 |
| Extrait d'ortie (*Urtica dioica*) | 84012-40-8 | 1 |
| Gomme xanthane | 11138-66-2 | 0,75 |
| Conservateur - extrait de pépins de pamplemousse | 90045-43-5 | 0,06 |

## Cocamidopropyl betaine (CAPB)

Fig. 1

## Sodium Lauryl Sulfate (SLS)

Fig. 2

Fig. 3

Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2306970 A1
- FR 2889057
- WO 200607922 A
- WO 0228402 A
- WO 03070260 A
- WO 2015000972 A1
- WO 2015019307 A1

- US 20080107699 A1
- WO 2004035885 A1
- WO 2013188626 A2
- US 20140065218 A1
- US 9157054 B2
- WO 2010045541 A1
- US 20150071977 A1

**Non-patent literature cited in the description**

- **WALKER WA.** Mechanisms of action of probiotics. *Clin Infect Dis.,* 2008, vol. 46 (2), S87-S91
- **REID, G. ; JASS, J. ; SEBULSKY, M. T. ; MCCORMICK, J. K.** Potential Uses of Probiotics in Clinical Practice. *Clinical Microbiology Reviews,* 2003, vol. 16 (4), 658-672
- **TSAO, R. ; YANG, R. ; YOUNG, J.C.** Antioxidant isoflavones in orange, maclura pomífera (Raf.) Scheneid. *J Agric Food Chem,* 2003, vol. 51, 6445-6451
- **WANG, Y.C. ; YU, R.C. ; CHOU, C-C.** Antioxidative activities of soymilk fermented with lactic acid bacteria and bifidobacteria. *Food Microbiol,* 2006, vol. 23, 128-135
- **T. HUQ.** *Encapsulation of Probiotic Bacteria in Biopolymeric System*
- Surface-Active Agents: Advances in Research and Application. 2011
- **VASILY N. DANILEVICH ; LADA E. PETROVSKAYA ; EUGENE V. GRISHIN.** A Highly Efficient Procedure for the Extraction of Soluble Proteins from Bacterial Cells with Mild Chaotropic Solutions. *Chem. Eng. Technol.,* 2008, vol. 31 (6), 904-910
- **RIAZ QU ; MASUD T.** Recent trends and applications of encapsulating materials for probiotic stability. *Crit Rev Food Sci Nutr.,* 2013, vol. 53 (3), 231-44
- *CHEMICAL ABSTRACTS,* 68891-38-3
- *CHEMICAL ABSTRACTS,* 68515-73-1
- *CHEMICAL ABSTRACTS,* 68603-42-9
- *CHEMICAL ABSTRACTS,* 56-81-5
- *CHEMICAL ABSTRACTS,* 5989-27-5
- *CHEMICAL ABSTRACTS,* 233799
- *CHEMICAL ABSTRACTS,* 9004-58-4

- *CHEMICAL ABSTRACTS,* 26172-55-4
- *CHEMICAL ABSTRACTS,* 2682-20-4
- *CHEMICAL ABSTRACTS,* 100-51-6
- *CHEMICAL ABSTRACTS,* 5949-29-1
- *CHEMICAL ABSTRACTS,* 68187-30-4
- *CHEMICAL ABSTRACTS,* 110615-47-9
- *CHEMICAL ABSTRACTS,* 66455-29-6
- *CHEMICAL ABSTRACTS,* 84696-25-3
- *CHEMICAL ABSTRACTS,* 90063-92-6
- *CHEMICAL ABSTRACTS,* 84012-40-8
- *CHEMICAL ABSTRACTS,* 11138-66-2
- *CHEMICAL ABSTRACTS,* 598-82-3
- *CHEMICAL ABSTRACTS,* 90045-43-5
- *CHEMICAL ABSTRACTS,* 77-92-9
- *CHEMICAL ABSTRACTS,* 141464-42-8
- **RIAZ QU ; MASUD T.** Recent trends and applications of encapsulating materials for probiotic stability. *Crit Rev Food Sci Nutr.,* 2013, vol. 53 (3), 231-44
- **M. ZHAO ; F. QU ; S. CAI ; Y. FANG ; K. NISHINARI ; G.O. PHILLIPS ; F. JIANG.** Microencapsulation of lactobacillus acidophilus CGMCC1. 2686: correlationbetween bacteria survivability and physical properties of microcapsules. *FoodBiophys.,* 2015, vol. 10, 292-299, https://doi.org/10.1007/s11483-014-9389-5
- **BORA, P.S. ; PURI, V. ; BANSAL, A.K.** Physicochemical Properties and Excipient Compatibility studies of Probiotic Bacillus coagulans Spores. *Sci. Pharm.,* 2009, vol. 77, 625-638, https://doi.org/10.3797/scipharm.0904-01
- **HUQ, T. ; KHAN. A. ; KHAN, R.A. ; RIEDL, B. ; LACROIX, M.** *Encapsulation of Probiotic Bacteria in Biopolymeric System, Critical Reviews in Food Science and Nutrition,* 2013, vol. 53 (9), 909-916